# EUROPÄISCHE PATENTSCHRIFT

(11) **EP 3 382 413 B1**
(45) Veröffentlichungstag und Bekanntmachung des Hinweises auf die Patenterteilung: **10.08.2022**
(21) Anmeldenummer: 17162995.9
(22) Anmeldetag: 27.03.2017
(51) Int. Cl.: G01R 33/48, G01R 33/567, G01R 33/561

(54) **2D NAVIGATORTECHNIK IN DER MRT**
2D NAVIGATOR TECHNIQUE IN MRI
TECHNIQUE DE NAVIGATION 2D DANS L'IRM

(43) Veröffentlichungstag der Anmeldung: 03.10.2018
(73) Patentinhaber: Siemens Healthcare GmbH, 91052 Erlangen (DE)
(72) Erfinder: Forman, Christoph, 91052 Erlangen (DE); Prochaska, Ivo, 71665 Vaihingen an der Enz (DE); Wetzl, Jens, 91080 Spardorf (DE)

(56) Entgegenhaltungen:
- PROCHASKA I ET AL: "Feasibility Study: 2-D Self-Navigation using Compressed Sensing Reconstruction for Respiratory Gating in Free-breathing 3-D CINE Imaging", INTERNATIONAL SOCIETY FOR MAGNETIC RESONANCE IN MEDICINE, ISMRM, 2030 ADDISON STREET, 7TH FLOOR, BERKELEY, CA 94704 USA, Nr. 3161, 7. April 2017 (2017-04-07), Seite 3161, XP040690729,
- MARKUS HENNINGSSON ET AL: "Whole-heart coronary MR angiography using image-based navigation for the detection of coronary anomalies in adult patients with congenital heart disease : Image-Navigated Coronary MRA", JOURNAL OF MAGNETIC RESONANCE IMAGING, Bd. 43, Nr. 4, 9. Oktober 2015 (2015-10-09), Seiten 947-955, XP055411293, US ISSN: 1053-1807, DOI: 10.1002/jmri.25058
- KEIGO KAWAJI ET AL: "Direct Coronary Artery Motion Tracking from Cartesian 2D Fat Image Navigator for Motion Corrected Coronary MRA", PROCEEDINGS OF THE INTERNATIONAL SOCIETY FOR MAGNETIC RESONANCE IN MEDICINE, ISMRM, 20TH ANNUAL MEETING AND EXHIBITION, MELBOURNE, AUSTRALIA, 5-11 MAY 2012, 21. April 2012 (2012-04-21), Seite 3813, XP040626234,
- MARKUS HENNINGSSON ET AL: "Prospective respiratory motion correction for coronary MR angiography using a 2D image navigator", MAGNETIC RESONANCE IN MEDICINE, Bd. 69, Nr. 2, 23. April 2012 (2012-04-23) , Seiten 486-494, XP55087203, ISSN: 0740-3194, DOI: 10.1002/mrm.24280

## Beschreibung

Die vorliegende Erfindung betrifft ein Verfahren zur Erstellung von atemkorrigierten MR-Bildern eines Untersuchungsvolumens einer Untersuchungsperson, bei einer Atembewegung der Untersuchungsperson, wobei das Untersuchungsvolumen das Herz beinhaltet. Die Erfindung betrifft weiterhin die zugehörige MR-Anlage, ein Computerprogrammprodukt und einen elektronisch lesbaren Datenträger.

Bei der Erstellung von 3D Bildern des Herzens im zeitlichen Verlauf, sogenannten CINE-Bildern, beträgt die gesamte Akquisitionszeit typischerweise zwischen drei und fünf Minuten. Diese Aufnahmen können nicht in der Atemanhaltetechnik aufgenommen werden, so dass die Untersuchungsperson während der Aufnahme der MR Daten frei atmet. Diese atembedingte Bewegung führt zu Artefakten in den rekonstruierten MR-Bildern. Für die dreidimensionale Bildgebung des Herzens in einer Herzphase, beispielsweise der Koronarangiographie, MRA, gibt es unterschiedliche Verfahren, die Atembewegung bei den Untersuchungen zu detektieren und zu kompensieren. Bei der Navigatortechnik wird die Atembewegung detektiert und kann dann bei der Berechnung der MR-Bilder berücksichtigt werden, um die Atembewegung zu kompensieren. Es sind externe Navigatoren bekannt, bei denen über eine externe Vorrichtung, wie beispielsweise einem Atemgurt oder anderen Vorrichtungen die Atembewegung detektiert wird und die Atembewegung dann entweder bei der Bildaufnahme oder bei der Bildrekonstruktion berücksichtigt wird. Weiterhin sind Bild- oder Eigennavigatortechniken bekannt, bei denen die MR-Bilder selber verwendet werden, um anhand der aufgenommenen MR-Signale auf die Atembewegung zu schließen.

Für eine dynamische 3D Bildgebung des Herzens müssen die MR-Signale kontinuierlich aufgenommen werden über den Herzzyklus, um Bilder zu den verschiedenen Zeitpunkten des Herzzyklus zu haben. Bei dieser kontinuierlichen Datenaufnahme über den Herzzyklus ist die Eigennavigatortechnik die Technik der Wahl, da dies die einzige Option ist, die Atmung zu detektieren wenn Wartezeiten für die Akquisition von speziellen Navigatordaten vermieden werden sollen. Für die dreidimensionale Darstellung des Herzens sind sogenannte 1D Eigennavigatortechniken bekannt, bei der eine Linie im Zentrum des Rohdatenraums periodisch in Superior-Inferiorrichtung, d.h. vom Kopf in Richtung der Füße periodisch aufgenommen wird. Jeder Punkt in der Fouriertransformation dieser Linie stellt eine Projektion einer axialen Ebene des Oberkörpers dar. Bei Messungen mit mehreren Empfangsspulen kann die Atembewegung auch detektiert werden durch Verwendung eines Bildes einer einzigen Spule anstelle der Kombination der Daten von allen Spulen. Diese einzelne Spule muss manuell ausgewählt werden durch Überprüfung von aufgenommenen Bilddaten, so dass die Rekonstruktion hier nicht vollständig automatisch abläuft. Diese eindimensionale Information kann dann zur Berechnung einer Atemkurve verwendet werden, um die Atemkurve bei der Aufnahme der eigentlichen MR-Bilddaten zu berücksichtigen, sei es durch sogenannte Gating-Funktionen, bei der nur Daten einer gewissen Atemphase berücksichtigt werden, oder durch andere Techniken, die die atembedingte Bewegung in den MR-Bildern korrigieren.

Aus den folgenden Schriften sind Methoden zur Akquisition von Navigatordaten im Rahmen der Herzbildgebung bekannt:
- Markus Henningsson ET AL: "Whole-heart coronary MR angiography using image-based navigation for the detection of coronary anomalies in adult patients with congenital heart disease: lmage-Navigated Coronary MRA", JOURNAL OF MAGNETIC RESONANCE IMAGING, Bd. 43, Nr. 4, 9. Oktober2015 (2015-10-09), Seiten 947-955, XP055411293, US ISSN: 1053-1807, DOI: 10.1002/jmri.25058
- KEIGO KAWAJI ET AL: "Direct Coronary Artery Motion Tracking from Cartesian 2D Fat Image Navigator for Motion Corrected Coronary MRA", PROCEEDINGS OF THE INTERNATIONAL SOCIETY FOR MAGNETIC RESONANCE IN MEDICINE, ISMRM, 20TH ANNUAL MEETING AND EXHIBITION, MELBOURNE, AUSTRALIA, 5-11 MAY 2012, 21. April 2012 (2012-04-21), Seite 3813, XP040626234
- Markus Henningsson ET AL: "Prospective respiratory motion correction for coronary MR angiography using a 2D image navigator", Magnetic Resonance in Medicine, Bd. 69, Nr. 2, 23. April 2012 (2012-04-23), Seiten 486-494, XP55087203, ISSN: 0740-3194, DOI: 10.1002/mrm. 24280

Es besteht die Aufgabe, die Atemkorrektur weiter zu verbessern und zu automatisieren, insbesondere die Detektion und Quantifizierung der Atembewegung zu verbessern.

Diese Aufgabe wird durch die Merkmale der unabhängigen Ansprüche gelöst. Weitere Merkmale sind in den abhängigen Ansprüchen beschrieben.

Gemäß einem ersten Aspekt wird ein Verfahren zur Erstellung von atemkorrigierten MR-Bildern eines Untersuchungsvolumens einer Untersuchungsperson gemäß Anspruch bereitgestellt, wobei die Untersuchungsperson atmet und das Untersuchungsvolumen das Herz umfasst. Es werden kontinuierlich Aufnahmen von MR-Signalen des Untersuchungsvolumens während mehrerer Herzzyklen erzeugt, wobei ein Herzzyklus mehrere Zeitabschnitte aufweist. Bei dieser kontinuierlichen Aufnahme wird pro Herzzyklus zumindest ein 2D Navigatorbilddatensatz während eines Zeitabschnitts des Herzzyklus aufgenommen, bei dem das Untersuchungsvolumen angeregt wird und ein Rohdatenraum in einem kartesischen Aufnahmemuster so mit MR-Signalen gefüllt wird, dass eine Ortsauflösung in zwei von drei Raumrichtungen des Untersuchungsvolumens erfolgt. Weiterhin werden mehrere 3D Bilddatensätze in den anderen Zeitabschnitten des Herzzyklus aufgenommen, wobei bei der Aufnahme der Bilddatensätze ein Rohdatenraum in einem kartesischen Aufnahmemuster so mit MR-Signalen gefüllt wird, dass eine Ortsauflösung in allen drei Raumrichtungen des Untersuchungsvolumens erfolgt. Anschließend erfolgt die Bestimmung der Atembewegung unter Verwendung der Navigatorbilddatensätze, die über die verschiedenen Herzzyklen aufgenommen wurden. Die atemkorrigierten MR-Bilder werden dann erzeugt durch Korrigieren der bestimmten Atembewegung in den aufgenommenen MR-Signalen. Die Korrektur der Atembewegung kann erfolgen durch das sogenannte "Gating", bei dem nur Bilder einer bestimmten Atemphase verwendet werden, oder die Atembewegung wird in den atemkorrigierten MR-Bildern wirklich korrigiert durch Entfernen der Atembewegung in den MR-Signalen.

Durch die Ortsauflösung in zwei der drei Dimensionen bei dem Navigatorbilddatensatz ist es möglich, verschiedene anatomische Bereiche und deren Bewegung genau zu detektieren. Durch die Verwendung von kartesischen Aufnahmemustern, in denen der Rohdatenraum kartesisch mit Rohdaten gefüllt wird, können schnelle und einfachere Bildrekonstruktionsverfahren verwendet werden. Durch die zweidimensionale Möglichkeit die Bewegung zu bestimmen kann auch eine Bewegungskompensation in zwei Richtungen verwendet werden. Jeder Punkt eines 2D Navigationsbildes kann eine Projektion einer rechts-links Linie des Oberkörpers sein.

Der Herzzyklus ist in mehrere Herzphasen unterteilt, wobei jede Herzphase mehrere Zeitabschnitte aufweist.

Die 2D Navigatorbilddatensätze werden alle während einer definierten Herzphase aufgenommen, beispielsweise der Diastole. Erfindungsgemäß erfolgt die Aufnahme der Navigatorbilddatensätze in unterschiedlichen Zeitabschnitten dieser Herzphase, beispielsweise der Diastole, wie es im Anspruch 1 definiert wird. Die Aufnahme der Navigatorbilddatensätze erfolgt somit nicht immer zu dem gleichen Zeitabschnitt während einer Herzphase, sondern verteilt über mehrere Zeitabschnitte dieser Herzphase. Dadurch kann vermieden werden, dass von einem Zeitabschnitt keine dreidimensionalen Bilddatensätze aufgenommen werden, die für die Erstellung eines Films auf der Grundlage der MR-Bilder notwendig sind.

Zur Aufnahme der 2D Navigationsdatensätze und der 3D Bilddatensätze kann eine 3D BSSFP (Balanced Steady State Free Precession) Bildgebungssequenz verwendet werden. Bei dieser Sequenz ergibt sich ein Gleichgewichtszustand in der Entwicklung der Magnetisierung, das MR-Signal relaxiert nicht vollständig zurück in den Ruhezustand. Die Navigatorbilddatensätze und die 3D Bilddatensätze können sich nur in der Ortsauflösung in der dritten Dimension unterscheiden, das Anregungsvolumen ist vorzugsweise identisch. Im vorliegenden Fall hat eine derartige Sequenz den Vorteil, dass nicht vollständig auf die volle Relaxation der angeregten Kernspins gewartet werden muss, bis eine Aufnahme der Navigatorbilddatensätze erfolgen kann.

Durch die zweidimensionalen Navigatorbilddatensätze ist es möglich, die Bewegung eines definierten anatomischen Bereichs oder verschiedener anatomischer Bereiche zu verwenden, um die Atembewegung zu bestimmen, beispielsweise die zweidimensionale Bewegung des Zwerchfells, der Brust oder der Leber. Bei der Aufnahme der 2D Navigatorbilddatensätze wird der Rohdatenraum vorzugsweise in eine der beiden Richtungen, in die eine Ortsauflösung erfolgt, nicht vollständig nach der Nyquistbedingung mit Rohdaten gefüllt. Ebenso wird der Rohdatenraum bei der Aufnahme der 3D Bilddatensätze in zwei der drei Raumrichtungen, in die eine Ortslösung erfolgt, nicht vollständig nach der Nyquistbedingung mit Rohdaten gefüllt. Die Bildrekonstruktion der MR-Bilder der Navigatorbilddatensätze und der Bilddatensätze erfolgt vorzugsweise mit der Compressed Sensing Technologie, die bei unterabgetasteten Daten verwendet werden kann, insbesondere dann, wenn ein Großteil der Bildpunkte nach Anwendung einer mathematischen Transformation der erzeugten MR-Bilder, z.B. der Wavelet-Transformation, keine starke Signalintensität hat.

Für die Berechnung der atemkorrigierten MR-Bilder, die im zeitlichen Verlauf dargestellt werden können, den sogenannten CINE-Bildern, können die Navigatorbilddatensätze und die Bilddatensätze verwendet werden. Dies bedeutet, dass auch die Navigatorbilddatensätze zur Bildrekonstruktion verwendet werden können und nicht nur für die Bestimmung der Bewegung.

Erfindungsgemäß ist der Herzzyklus in eine festgelegte Anzahl von Zeitabschnitten unterteilt sein und in jedem der Zeitabschnitte wird entweder ein 2D Navigatorbilddatensatz oder ein 3D Bilddatensatz aufgenommen. Die Navigatorbilddatensätze sind hierbei so über die verschiedenen Herzzyklen auf die Zeitabschnitte der definierten Herzphasen verteilt, dass zu jedem Zeitabschnitt der definierten Herzphase auch 3D Bilddatensätze aufgenommen werden. Dies bedeutet, dass vorzugsweise pro Herzzyklus ein Navigatorbilddatensatz und in allen anderen Zeitabschnitten 3D Bilddatensätze aufgenommen werden, wobei jedoch erfindungsgemäß die Navigatorbilddatensätze in Bezug auf den Herzzyklus nicht immer im gleichen Zeitabschnitt aufgenommen werden.

Die Erfindung betrifft ebenso die in Anspruch 9 definierte, zugehörige MR-Anlage, die eine Steuereinheit und eine Speichereinheit aufweist. Die Speichereinheit kann von der Steuereinheit ausführbare Steuerinformationen speichern, wobei die MR-Anlage ausgebildet ist, bei Ausführungen der Steuerinformationen in der Steuereinheit die oben beschriebenen Schritte auszuführen, wenn die Steuerinformationen in der Steuereinrichtung ausgeführt werden.

Zusätzlich betrifft die Erfindung das in Anspruch 11 definierte Computerprogrammprodukt und den in Anspruch 12 definierten elektronisch lesbaren Datenträger.

Die oben beschriebenen Merkmale sowie die nachfolgend beschriebenen Merkmale können nicht nur in den entsprechend explizit dargestellten Kombinationen verwendet werden, sondern auch in anderen Kombinationen, sofern es nicht explizit anders erwähnt ist und hierdurch der durch die Ansprüche definierte Schutzbereich der Erfindung nicht verlassen wird.

Weiterhin ist es möglich, die verschiedenen Merkmale einzeln zu verwenden, sofern hierdurch der durch die Ansprüche definierte Schutzbereich der Erfindung nicht verlassen wird.

Die Erfindung wird unter Bezugnahme auf die beiliegenden Zeichnungen näher erläutert.
Figur 1 zeigt schematisch eine MR-Anlage, mit der erfindungsgemäß die atemkorrigierten MR-Bilder eines Herzens aufgenommen werden können.
Figur 2 zeigt schematisch die Aufteilung des Herzzyklus in mehrere Zeitabschnitte und die Aufteilung des Herzzyklus für die Aufnahme von Bilddatensätzen und Navigatorbilddatensätzen.
Figur 3 zeigt schematisch ein Aufnahmemuster für zwei verschiedene Herzphasen des Herzzyklus im Rohdatenraum, wobei in der einen Herzphase nur Bilddatensätze und in der anderen Herzphase Bilddatensätze und Navigatorbilddatensätze aufgenommen werden.
Figur 4 zeigt schematisch ein Flussdiagramm mit den Schritten, die bei der Bestimmung der atemkorrigierten MR-Bilder des Herzens in der MR-Anlage von Figur 1 durchgeführt werden.

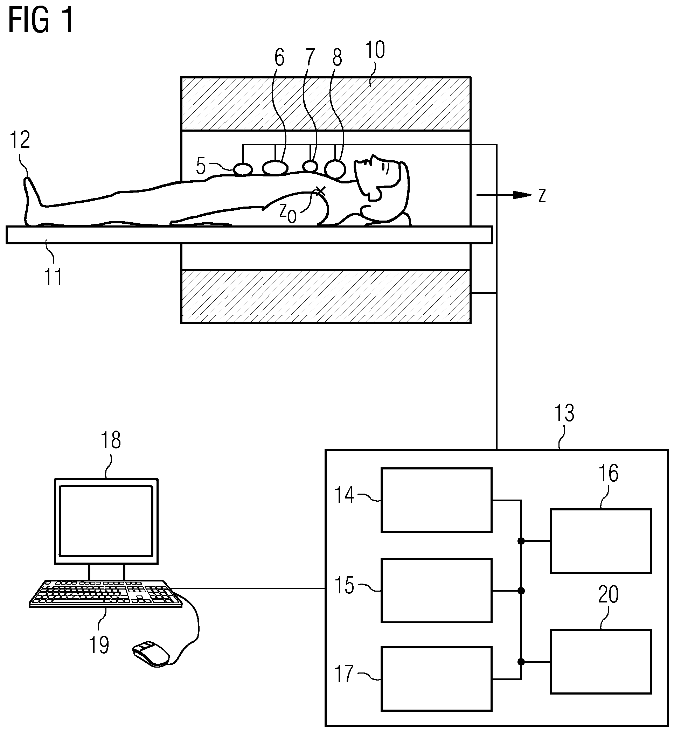

Figur 1 zeigt schematisch eine MR-Anlage, mit der erfindungsgemäß atemkorrigierte MR-Bilder einer Untersuchungsperson aufgenommen werden können. Die Magnetresonanzanlage weist einen Magneten 10 zur Erzeugung eines Polarisationsfeldes B0 auf, wobei eine auf der Liege 11 angeordnete Untersuchungsperson 12 das Untersuchungsobjekt darstellt, das in ein Isozentrum Z0 des Magneten 10 gefahren wird, um dort ortskodierte Magnetresonanzsignale des Untersuchungsobjekts aufzunehmen. Durch Einstrahlen von Hochfrequenzpulsen und Schalten von Magnetfeldgradienten kann die durch das Polarisationsfeld B0 erzeugte Magnetisierung gestört werden durch Auslenken der Kernspins aus der Gleichgewichtslage. Die bei der Rückkehr in die gleiche Rechtslage in Empfangsspulen 5 bis 8 induzierten Ströme können in Magnetresonanzsignale umgewandelt werden. Die allgemeine Funktionsweise zur Erstellung von MR-Bildern mit der Detektion von Magnetresonanzsignalen ist dem Fachmann bekannt, so dass auf eine detaillierte Beschreibung der Funktionsweise der MR-Anlage verzichtet wird.

Die Untersuchungsanlage weist eine Steuereinheit 13 auf, die zur Steuerung der MR-Anlage verwendet wird. Die Steuereinheit 13 weist eine Gradientensteuerung 14 und eine HF-Steuerung 15 zur Schaltung und Erzeugung der HF-Pulse zur Auslenkung der Kernspins aus der Gleichgewichtslage auf. Die HF-Einheit kann eine Multikanal-HF-Einheit sein oder eine Einkanal-HF-Einheit. In einer Speichereinheit 16 können beispielsweise die für die Aufnahme der MR-Bilder notwendigen Bildgebungssequenzen abgespeichert werden sowie alle weiteren Steuerinformationen, die notwendig sind, um die Erfindung auszuführen. Eine Bildsequenzsteuerung 17 steuert die Bildaufnahme und damit in Abhängigkeit von der gewählten Bildgebungssequenz die Abfolge der Magnetfeldgradienten, der HF-Pulse und der Empfangsintervalle der MR-Signale. Damit steuert die Bildsequenzsteuerung 17 auch die Gradientensteuerung 14 und die HF-Steuereinheit 15. In einer Recheneinheit 20 können MR-Bilder berechnet werden, die auf einer Anzeige 18 angezeigt werden können. Eine Bedienperson kann über eine Eingabeeinheit 19 die MR-Anlage steuern. Insbesondere die Recheneinheit 20 und die Bildsequenzsteuerung 17 können so ausgebildet sein, dass die 2D Navigatorbilddatensätze erzeugt werden, wobei die Recheneinheit 20 dann die Atembewegung identifiziert und quantifiziert und atemkorrigierte MR-Bilder berechnet. Die Recheneinheit 20 kann einen oder mehrere Prozessoren aufweisen, die in der Speichereinheit 16 gespeicherte Steuerinformationen verarbeitet.

Die in Figur 1 gezeigten Verbindungen zwischen den einzelnen funktionellen Einheiten kann drahtgebunden erfolgen, oder drahtlos.

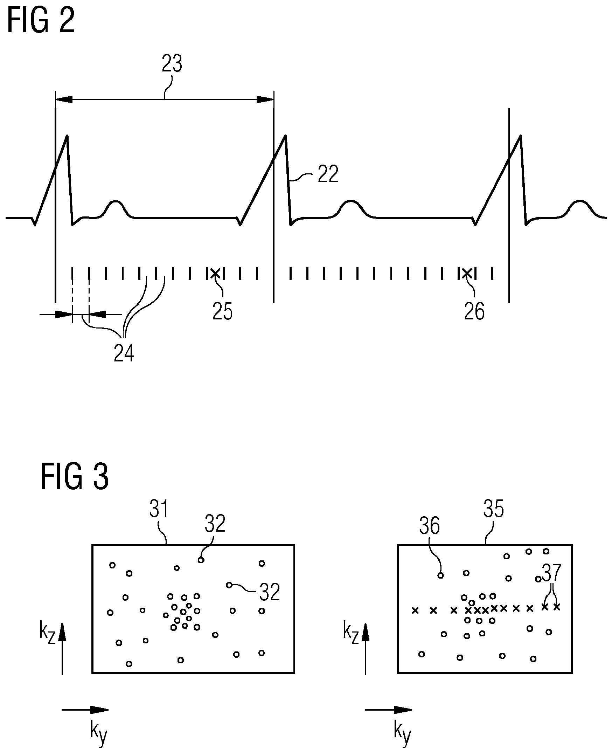

In Figur 2 ist schematisch ein zeitlicher Verlauf 22 der Herztätigkeit dargestellt. Ein Herzzyklus 23 ist in mehrere Zeitabschnitte 24 unterteilt. Wie nachfolgend noch näher erläutert werden wird, wird während jedem dieser Zeitabschnitte entweder ein 3D Bilddatensatz oder ein 2D Navigatorbilddatensatz aufgenommen. Die Zeitabschnitte, in denen ein Navigatorbilddatensatz aufgenommen wird, sind mit einem Kreuz dargestellt, in Figur 2 die Zeitabschnitte 25 und 26. Wie aus dem Vergleich des ersten Herzzyklus mit dem zweiten dargestellten Herzzyklus zu erkennen ist, erfolgt die Aufnahme des Navigatorbilddatensatzes nicht im zeitlichen Verlauf im gleichen Zeitabschnitt, sondern in verschiedenen Zeitabschnitten einer Herzphase, hier der Diastole. Erfindungsgemäß erfolgt die Aufnahme derart, dass eine kontinuierliche Aufnahme der MR-Signale erfolgt. Wenn der Herzzyklus beispielsweise in 20 Zeitabschnitte unterteilt ist, so werden in 19 Zeitabschnitten die 3D Bilddatensätze aufgenommen und in einem Zeitabschnitt die Navigatorbilddatensätze. In allen Zeitabschnitten außerhalb der Zeitabschnitte 25 und 26 werden die 3D Bilddatensätze aufgenommen.

Die 2D Bilddatensätze werden mithilfe einer BSSFP-Sequenz aufgenommen, beispielsweise während dem Ende der diastolischen Phase des Herzzyklus. Bei dieser zweidimensionalen kartesischen Aufnahme des Rohdatenraums wird ein MR-Bild erstellt, wobei nur die Auflösung in die dritte Dimension des Anregungsvolumens akkumuliert dargestellt ist. Die beiden Raumrichtungen, in denen eine Ortsauflösung erfolgt, kann die Arterior-Posteriorrichtung sein und die Superior-Inferiorrichtung, so dass nur eine Mittelung der MR-Signale über die dritte Dimension die laterale Richtung vorliegt. Dadurch ist es beispielsweise möglich, die Position des Brustkorbs und seine Bewegung zweidimensional festzustellen, was bei eindimensionalen Navigatordaten nicht möglich ist. Selbstverständlich können auch weitere Organe oder anatomische Bereiche wie die Leber oder das Zwerchfell und deren zweidimensionale Bewegung verwendet werden zur Bestimmung der Atembewegung.

In Figur 3 ist schematisch der Rohdatenraum für zwei verschiedene Herzphasen dargestellt, wie er mit Rohdaten gefüllt ist. Auf der linken Seite ist der Rohdatenraum 31 dargestellt, der den Rohdatenraum während der systolischen Herzphase zeigt. Jeder Rohdatenpunkt 32 entspricht einer aufgenommenen Rohdatenlinie, die in die Zeichenebene hinein verläuft, hier die Kx Richtung. Die Datenaufnahme erfolgt hierbei derart, dass die Rohdatenpunkte den 3D Bilddatensatz erzeugen, wobei in der dargestellten Raumrichtung Kz und Ky eine Unterabtastung erfolgt, so dass in diese beiden Richtungen Kz, Ky die Nyquistbedingung für eine artefaktfreie Rekonstruktion nicht erfüllt ist. Die Rekonstruktion der 3D Bilddatensätze kann mithilfe der Compressed Sensing Technologie erfolgen. Auf der rechten Seite von Figur 3 ist der Rohdatenraum 35 während einer diastolischen Phase dargestellt. Hier werden wiederum die Bilddatensätze mit den Rohdatenpunkten 36 bezeichnet, während die Rohdatenpunkte 37 die Rohdatenpunkte bezeichnen, bei denen die Navigatorbilddatensätze aufgenommen werden. Wie im rechten Bild von Figur 3 zu erkennen ist, werden die Akquisitionen für den Navigatorbilddatensatz über die verschiedenen Zeitabschnitte der diastolischen Herzphase verteilt, so dass unterschiedliche Bilddatensätze für Kz ungleich null vorhanden sind. Wie in Figur 3 im Rohdatensatz 35 zu erkennen ist, erfolgen alle Navigatorbilddatenaufnahmen bei Kz gleich null, so dass keine Ortsauflösung in diese dritte Raumrichtung vorhanden ist. Auch dieser 2D Projektionsdatensatz ist in die eine Raumrichtung, hier Ky, unterabgetastet und kann beispielsweise mithilfe der TPAT (Temporal Parallel Acquisition Technique) aufgenommen werden.

Die Datenrekonstruktion kann dann mithilfe der Compressed Sensing Technologie in zwei Schritten rekonstruiert werden. In einem ersten Schritt werden aus der kontinuierlichen Datenaufnahme die Navigatorbilddatensätze herausgefiltert, beziehungsweise diese werden bestimmt. Diese Datensätze werden dann rekonstruiert und zur Bestimmung der Bewegung verwendet.

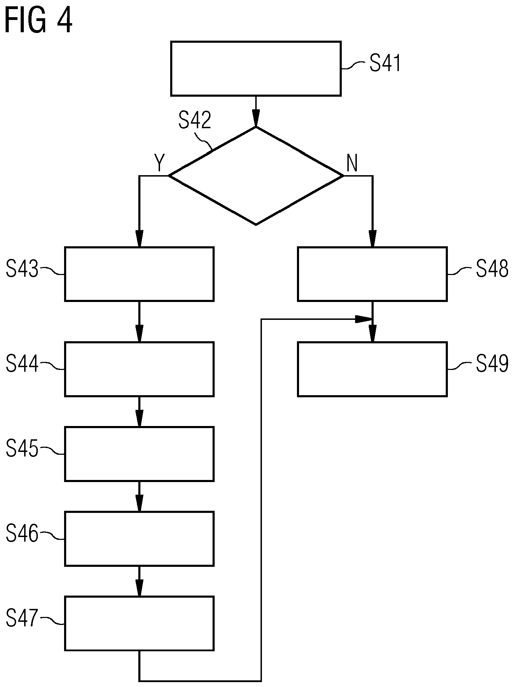

Das Verfahren zur Erstellung der atemkorrigierten MR-Bilder wird in Zusammenhang mit Figur 4 noch einmal näher erläutert. In einem Schritt S 41 erfolgt eine kontinuierliche Signalaufnahme. In einem Schritt S 42 wird dann geprüft, ob es sich um einen ersten Durchlauf bei dem Verfahren handelt. Wenn dies der Fall ist, werden in einem Schritt S 43 die Navigatorbilddatensätze in der kontinuierlichen Signalaufnahme bestimmt. Wie in Zusammenhang mit Figur 2 und 3 erläutert, unterscheiden sich die Navigatorbilddatensätze von den Bilddatensätzen dadurch, dass sie nur in zwei Raumrichtungen ortsaufgelöst sind, anstelle in drei Raumrichtungen, wie die Bilddatensätze. Anschließend erfolgt im Schritt S 44 die Rekonstruktion der Navigatorbilddatensätze. In einem Schritt S 45 wird dann der oder die anatomischen Bereiche identifiziert, anhand derer die Atembewegung bestimmt werden soll. Die Bewegungsdetektion kann auf verschiedene Arten bestimmt werden. Beispielsweise ist es möglich, das Herz als interessierende Region festzulegen und anschließend eine Registrierung über alle Bilder zu einem Referenznavigatorbilddatensatz zu machen.

Hierbei ist zu erwähnen, dass das Untersuchungsvolumen erfindungsgemäß das Herz beinhaltet. Mit den Bewegungsfeldern, die durch die Registrierung bestimmt wurden, kann der Mittelwert für die Größe der Bewegung berechnet werden und wenn die Bewegung kleiner als ein bestimmter Grenzwert ist, können die Daten dieses Zeitabschnitts für die Rekonstruktion der atemkorrigierten MR-Bilder verwendet werden.

Weiterhin kann die Atembewegung bestimmt werden durch Suche nach Ähnlichkeiten in der Struktur in den Navigatorbilddatensätzen. Bei diesem unter dem Namen Structural Similarity (SSIM) bekannten Verfahren werden die gesamten MR-Bilder oder Teile davon miteinander verglichen und die Daten werden verwendet, wenn der Ähnlichkeitswert größer als ein bestimmter Grenzwert ist. Eine andere Möglichkeit besteht darin, das Herz in allen Navigatorbilddatensätzen zu segmentierten. Anschließend wird der Schwerpunkt des Herzens als Referenzort gewählt und alle Aufnahmen der 3D Bilddatensätze verwendet, wo der Schwerpunkt an dem gleichen Ort liegt.

Nach dieser Bestimmung der Atembewegung in Schritt S 46 erfolgt in Schritt S 47 ein Filtern der 2D Navigatorbilddatensätze und der 3D Bilddatensätze nach der Atemphase, wenn nur Bilder einer einzelnen Atemphase berücksichtigt werden sollen (Schritt S 47). Dieses Filtern in Abhängigkeit von der Atemphase ist jedoch nicht zwingend notwendig, es können auch Bilder von allen Atemphasen berücksichtigt werden.

Wenn in Schritt S 42 festgestellt wurde, dass die Atembewegung im ersten Durchlauf bestimmt wurde, so können wiederum in Schritt S 48 die aufgenommenen MR-Datensätze nach der Atemphase gefiltert werden, um nur eine bestimmte Atemphase zu berücksichtigen, bevor in einem Schritt S 49 die atemkorrigierten MR-Signale erstellt werden.

Das oben beschriebene Verfahren hat den Vorteil, dass der ganze Oberkörper mit Lunge, Leber und Herz im Navigatorbilddatensatz dargestellt werden kann, so dass die Position des Herzens gut überwacht werden kann. Es ermöglicht eine Bewegungskompensation in zwei Richtungen. Ein weiterer Vorteil besteht darin, dass das oben beschriebene Verfahren vollständig automatisiert werden kann. Eine Interaktion mit einer Person der MR-Anlage ist nicht zwingend notwendig.

## Patentansprüche

1. Verfahren zur Erstellung von atemkorrigierten MR-Bildern eines Untersuchungsvolumens einer Untersuchungsperson bei einer Atembewegung der Untersuchungsperson (12), wobei das Untersuchungsvolumen das Herz beinhaltet, mit den folgenden Schritten:
- kontinuierliches Aufnehmen von MR-Signalen des Untersuchungsvolumens während mehrerer Herzzyklen (23), wobei ein Herzzyklus (23) mehrere Zeitabschnitte(24, 25, 26) aufweist und in mehrere Herzphasen unterteilt ist, wobei jede Herzphase mehrere Zeitabschnitte aufweist, wobei das kontinuierliche Aufnehmen eine Aufnahme von einem 2D Navigatorbilddatensatz pro Herzzyklus (23) während eines Zeitabschnittes (24, 25, 26) des Herzzyklus (23) aufweist, bei dem das Untersuchungsvolumen angeregt wird und ein Rohdatenraum in einem kartesischen Aufnahmemuster so mit MR-Signalen gefüllt wird, dass eine Ortsauflösung in zwei von drei Raumrichtungen des Untersuchungsvolumens erfolgt, wobei in einem ersten Herzzyklus (23) der mehreren Herzzyklen (23) die Aufnahme des 2D Navigatorbilddatensatzes in einem ersten Zeitabschnitt (25) einer Herzphase erfolgt und in einem zweiten Herzzyklus der mehreren Herzzyklen die Aufnahme des 2D Navigatorbilddatensatzes in einem zweiten Zeitabschnitt (26) derselben Herzphase erfolgt, wobei der erste Zeitabschnitt (25) verschieden vom zweiten Zeitabschnitt (26) ist,
wobei die kontinuierliche Aufnahme eine Aufnahme von mehreren 3D Bilddatensätzen in den anderen Zeitabschnitten (24) des Herzzyklus (23) aufweist, wobei bei der Aufnahme der Bilddatensätze ein Rohdatenraum in einem kartesischen Aufnahmemuster so mit MR-Signalen gefüllt wird, dass eine Ortsauflösung in allen drei Raumrichtungen des Untersuchungsvolumens erfolgt,
- Bestimmen der Atembewegung anhand der Navigatorbilddatensätze, die in den mehreren Herzzyklen (23) aufgenommen wurden,
- Korrigieren der bestimmten Atembewegung in den aufgenommenen MR-Signalen zur Erstellung der atemkorrigierten MR-Bilder.

2. Verfahren nach Anspruch 1, wobei die 2D Navigatorbilddatensätze alle während einer definierten Herzphase aufgenommen werden, jedoch in unterschiedlichen Zeitabschnitten (24, 25, 26) dieser Herzphase.

3. Verfahren nach Anspruch 2, **dadurch gekennzeichnet, dass** die 2D Navigatorbilddatensätze während unterschiedlicher Zeitabschnitte (24, 25, 26) der Diastole des Herzens gemessen werden.

4. Verfahren nach einem der vorstehenden Ansprüche, **dadurch gekennzeichnet, dass** zur Aufnahme der 2D Navigatorbilddatensätze und der 3D Bilddatensätze eine 3D Balanced Steady State Free Prescession BSSFP-Sequenz verwendet wird.

5. Verfahren nach einem der vorstehenden Ansprüche, **dadurch gekennzeichnet, dass** die Atembewegung bestimmt wird durch Bestimmung einer Bewegung von zumindest einem definierten anatomischen Bereich, der in den 2D Navigatorbilddatensätzen identifiziert worden ist.

6. Verfahren nach einem der vorstehenden Ansprüche, **dadurch gekennzeichnet, dass** bei der Aufnahme der 2D Navigatorbilddatensätze der Rohdatenraum in eine der beiden Richtungen, in die eine Ortsauflösung erfolgt, nicht vollständig nach der Nyquistbedingung mit Rohdaten gefüllt wird, und der Rohdatenraum bei der Aufnahme der 3D Bilddatensätze in zwei der drei Raumrichtungen, in die eine Ortsauflösung erfolgt, nicht vollständig nach der Nyquistbedingung mit Rohdaten gefüllt wird, wobei eine Bildrekonstruktion von MR-Bildern der Navigatordatensätze und der Bilddatensätze mit der Compressed Sensing Technologie erfolgt.

7. Verfahren nach einem der vorstehenden Ansprüche, **dadurch gekennzeichnet, dass** die atemkorrigierten MR-Bilder einen zeitlichen Verlauf der Herzbewegung darstellen und unter Verwendung der Navigatorbilddatensätze und der Bilddatensätze berechnet werden.

8. Verfahren nach einem der vorstehenden Ansprüche, **dadurch gekennzeichnet, dass** die Atembewegung für die zwei Raumrichtungen der Navigatorbilddatensätze bestimmt und korrigiert wird.

9. MR-Anlage (10), die ausgebildet ist zur Erstellung von atemkorrigierten MR-Bildern eines Untersuchungsvolumens einer Untersuchungsperson bei einer Atembewegung der Untersuchungsperson, wobei das Untersuchungsvolumen das Herz beinhaltet, wobei die MR-Anlage eine Steuereinheit (13) und eine Speichereinheit (16) aufweist, wobei die Speichereinheit von der Steuereinheit ausführbare Steuerinformationen speichert, wobei die MR-Anlage ausgebildet ist, bei Ausführung der Steuerinformationen in der Steuereinheit folgende Schritte auszuführen:
- kontinuierliches Aufnehmen von MR-Signalen des Untersuchungsvolumens während mehrerer Herzzyklen (23), wobei ein Herzzyklus (23) mehrere Zeitabschnitte (24, 25, 26) aufweist und in mehrere Herzphasen unterteilt ist, wobei jede Herzphase mehrere Zeitabschnitte aufweist, wobei das kontinuierliche Aufnehmen eine Aufnahme von einem 2D Navigatorbilddatensatz pro Herzzyklus (23) während eines Zeitabschnittes (24, 25, 26) des Herzzyklus (23) aufweist, bei dem das Untersuchungsvolumen angeregt wird und ein Rohdatenraum in einem kartesischen Aufnahmemuster so mit MR-Signalen gefüllt wird, dass eine Ortsauflösung in zwei von drei Raumrichtungen des Untersuchungsvolumens erfolgt, wobei in einem ersten Herzzyklus (23) der mehreren Herzzyklen (23) die Aufnahme des 2D Navigatorbilddatensatzes in einem ersten Zeitabschnitt (25) einer Herzphase erfolgt und in einem zweiten Herzzyklus der mehreren Herzzyklen die Aufnahme des 2D Navigatorbilddatensatzes in einem zweiten Zeitabschnitt (26) derselben Herzphase erfolgt, wobei der erste Zeitabschnitt (25) verschieden vom zweiten Zeitabschnitt (26) ist, wobei die kontinuierliche Aufnahme eine Aufnahme von mehreren 3D Bilddatensätzen in den anderen Zeitabschnitten (24) des Herzzyklus (23) aufweist, wobei bei der Aufnahme der Bilddatensätze ein Rohdatenraum in einem kartesischen Aufnahmemuster so mit MR-Signalen gefüllt wird, dass eine Ortsauflösung in allen drei Raumrichtungen des Untersuchungsvolumens erfolgt,
- Bestimmen der Atembewegung anhand der Navigatorbilddatensätze, die in den mehreren Herzzyklen (23) aufgenommen wurden,
- Korrigieren der bestimmten Atembewegung in den aufgenommenen MR-Signalen zur Erstellung der atemkorrigierten MR-Bilder.

10. MR-Anlage nach Anspruch 9, **dadurch gekennzeichnet, dass** die MR-Anlage ausgebildet ist, bei Ausführung der Steuerinformationen in der Steuereinheit (13) ein Verfahren nach einem der Ansprüche 2 bis 8 auszuführen.

11. Computerprogrammprodukt, welches Programmmittel umfasst und direkt in eine Speichereinheit (16) einer programmierbaren Steuereinheit (13) einer MR-Anlage ladbar ist, um alle Schritte des Verfahrens nach einem der Ansprüche 1 bis 8 auszuführen, wenn die Programmmittel in der Steuereinrichtung ausgeführt werden.

12. Elektronisch lesbarer Datenträger mit darauf gespeicherten elektronisch lesbaren Steuerinformationen, welche derart ausgestaltet sind, dass sie bei Verwendung des Datenträgers in einer Steuereinheit (13) einer MR-Anlage das Verfahren nach einem der Ansprüche 1 bis 8 durchführen, wenn die Steuerinformationen in der Steuereinheit ausgeführt werden.

## Claims

1. Method for producing respiration-corrected MR images of an examination volume of an examined person while respiratory movement of the examined person (12) is present, wherein the examination volume contains the heart, comprising the following steps:
- continuously recording MR signals of the examination volume during a plurality of cardiac cycles (23), wherein a cardiac cycle (23) comprises a plurality of time segments (24, 25, 26), wherein each cardiac phase has a plurality of time segments,
wherein the continuous recording includes a recording of one 2D navigator image data record per cardiac cycle (23) during a time segment (24, 25, 26) of the cardiac cycle (23), as part of which the examination volume is excited and a raw data space in a Cartesian recording model is filled with MR signals in such a way that a spatial resolution is achieved in two of three spatial directions of the examination volume, wherein, in a first cardiac cycle (23) of the plurality of cardiac cycles (23), the recording of the 2D navigator image data record is achieved in a first time segment (25) of a cardiac phase and, in a second cardiac cycle of the plurality of cardiac cycles, the recording of the 2D navigator image data record is achieved in a second time segment (26) of the same cardiac phase, wherein the first time segment (25) is different from the second time segment (26),
wherein the continuous recording includes a recording of a plurality of 3D image data records in the other time segments (24) of the cardiac cycle (23), wherein as part of the recording of said image data records a raw data space in a Cartesian recording model is filled with MR signals in such a way that a spatial resolution is achieved in all three spatial directions of the examination volume,
- determining the respiratory movement on the basis of the navigator image data records which were recorded in the plurality of cardiac cycles (23),
- correcting the determined respiratory movement in the recorded MR signals in order to produce the respiration-corrected MR images.

2. Method according to claim 1, wherein the 2D navigator image data records are all recorded during a defined cardiac phase but in different time segments (24, 25, 26) of this cardiac phase.

3. Method according to claim 2, **characterised in that** the 2D navigator image data records are measured during different time segments (24, 25, 26) of the diastole of the heart.

4. Method according to one of the preceding claims, **characterised in that** a 3D Balanced Steady State Free Precession BSSFP sequence is used for the purpose of recording the 2D navigator image data records and the 3D image data records.

5. Method according to one of the preceding claims, **characterised in that** the respiratory movement is determined by means of determining a movement of at least one defined anatomical region which has been identified in the 2D navigator image data records.

6. Method according to one of the preceding claims, **characterised in that** when the 2D navigator image data records are recorded, the raw data space in one of the two directions in which a spatial resolution is achieved is not completely filled with raw data as per the Nyquist criterion, and when the 3D image data records are recorded, the raw data space in two of the three spatial directions in which a spatial resolution is achieved is not completely filled with raw data as per the Nyquist criterion, wherein an image reconstruction of MR images of the navigator data records and the image data records is performed using Compressed Sensing Technology.

7. Method according to one of the preceding claims, **characterised in that** the respiration-corrected MR images represent a temporal profile of the heart movement and are calculated using the navigator image data records and the image data records.

8. Method according to one of the preceding claims, **characterised in that** the respiratory movement is determined and corrected for the two spatial directions of the navigator image data records.

9. MR installation (10) which is designed for the purpose of producing respiration-corrected MR images of an examination volume of an examined person while respiratory movement of the examined person is present, wherein the examination volume contains the heart, wherein the MR installation comprises a control unit (13) and a memory unit (16), wherein the memory unit stores control information that can be executed by the control unit, wherein in the context of executing the control information in the control unit the MR installation is designed to execute the following steps:
- continuously recording MR signals of the examination volume during a plurality of cardiac cycles (23), wherein a cardiac cycle (23) comprises a plurality of time segments (24, 25, 26) and is divided into a plurality of cardiac phases, wherein each cardiac phase has a plurality of time segments,
wherein the continuous recording includes a recording of one 2D navigator image data record per cardiac cycle (23) during a time segment (24, 25, 26) of the cardiac cycle (23), as part of which the examination volume is excited and a raw data space in a Cartesian recording model is filled with MR signals in such a way that a spatial resolution is achieved in two of three spatial directions of the examination volume, wherein, in a first cardiac cycle (23) of the plurality of cardiac cycles (23), the recording of the 2D navigator image data record is achieved in a first time segment (25) of a cardiac phase and, in a second cardiac cycle of the plurality of cardiac cycles, the recording of the 2D navigator image data record is achieved in a second time segment (26) of the same cardiac phase, wherein the first time segment (25) is different from the second time segment (26),
wherein the continuous recording includes a recording of a plurality of 3D image data records in the other time segments (24) of the cardiac cycle (23), wherein as part of the recording of said image data records a raw data space in a Cartesian recording model is filled with MR signals in such a way that a spatial resolution is achieved in all three spatial directions of the examination volume,
- determining the respiratory movement on the basis of the navigator image data records which were recorded in the plurality of cardiac cycles (23),
- correcting the determined respiratory movement in the recorded MR signals in order to produce the respiration-corrected MR images.

10. MR installation according to claim 9, **characterised in that** in the context of executing the control information in the control unit (13), the MR installation is designed to execute a method according to one of the claims 2 to 8.

11. Computer program product which comprises program means and can be loaded directly into a memory unit (16) of a programmable control unit (13) of an MR installation, in order to execute all steps of the method according to one of the claims 1 to 8 when the program means are executed in the control device.

12. Electronically readable data medium on which is stored electronically readable control information, this being so configured as to perform the method according to one of the claims 1 to 8 when the data medium is used in a control unit (13) of an MR installation when the control information is executed in the control unit.

## Revendications

1. Procédé de création d'images RM corrigées de respiration d'un volume à examiner d'une personne à examiner, lors d'un mouvement respiratoire de la personne (12) à examiner, dans lequel le volume à examiner contient le cœur, ayant les stades suivants :
- enregistrement continu de signaux RM du volume à examiner pendant plusieurs cycles (23) cardiaques, dans lequel un cycle (23) cardiaque a plusieurs laps de temps (24, 25, 26) et est subdivisé en plusieurs phases cardiaques, dans lequel chaque phase cardiaque a plusieurs laps de temps,
dans lequel l'enregistrement continu comporte un enregistrement d'un ensemble de données d'image de navigation en 2D par cycle (23) cardiaque, pendant un laps de temps (24, 25, 26) du cycle (23) cardiaque, dans lequel on excite le volume à examiner et on remplit un espace de données brutes dans un modèle d'enregistrement cartésien de signaux RM, de manière à ce qu'une résolution spatiale dans deux de trois directions de l'espace du volume à examiner ait lieu, dans lequel, dans un premier cycle (23) cardiaque des plusieurs cycles (23) cardiaques, l'enregistrement de l'ensemble de données d'image de navigation en 2D a lieu dans un premier laps de temps (25) d'une phase cardiaque et, dans un deuxième cycle cardiaque des plusieurs cycles cardiaques, l'enregistrement de l'ensemble de données d'image de navigation en 2D a lieu dans une deuxième laps de temps (26) de la même phase cardiaque, dans lequel le premier laps de temps (25) est différent du deuxième laps de temps (26), dans lequel l'enregistrement continu comporte un enregistrement de plusieurs ensembles de données d'image en 3D dans les autres laps de temps (24) du cycle (23) cardiaque, dans lequel, lors de l'enregistrement des ensembles de données d'image, on remplit un espace de données brutes dans un modèle d'enregistrement cartésien de signaux MR, de manière à ce qu'une résolution spatiale dans toutes les trois directions de l'espace du volume à examiner ait lieu,
- détermination du mouvement respiratoire à l'aide des ensembles de données d'image de navigation, qui ont été enregistrés dans les plusieurs cycles (23) cardiaques,
- correction du mouvement respiratoire déterminé dans les signaux RM enregistrés pour la création des images RM corrigées de respiration.

2. Procédé suivant la revendication 1, dans lequel on enregistre les ensembles de données d'image de navigation en 2D, tous pendant une phase cardiaque définie, toutefois dans des laps de temps (24, 25, 26) différents de cette phase cardiaque.

3. Procédé suivant la revendication 2, **caractérisé en ce que** l'on mesure les ensembles de données d'image de navigation en 2D pendant des laps de temps (24, 25, 26) différents de la diastole du cœur.

4. Procédé suivant l'une des revendications précédentes, **caractérisé en ce que**, pour l'enregistrement des ensembles de données d'image de navigation en 2D et des ensembles de données d'image en 3D, on utilise une séquence Balanced Steady State Free Prescession BSSFP en 3D.

5. Procédé suivant l'une des revendications précédentes, **caractérisé en ce que** l'on détermine le mouvement respiratoire par détermination d'un mouvement d'au moins une partie anatomique définie, qui a été identifiée dans les ensembles de données d'image de navigation en 2D.

6. Procédé suivant l'une des revendications précédentes, **caractérisé en ce que**, lors de l'enregistrement des ensembles de données d'image de navigation en 2D, on ne remplit pas complètement, suivant le critère de Nyquist, de données brutes l'espace de données brutes dans l'une des deux directions, dans lesquelles une résolution spatiale a lieu, et on ne remplit pas complètement de données brutes, suivant le critère de Nyquist, l'espace de données brutes, lors de l'enregistrement des ensembles de données d'image en 3D dans deux des trois directions de l'espace, dans lesquelles une résolution spatiale a lieu, une reconstruction d'images RM des ensembles de données de navigation et des ensembles de données d'image ayant lieu par la compressed sensing technologie.

7. Procédé suivant l'une des revendications précédentes, **caractérisé en ce que** les images RM corrigées de respiration représentent une courbe en fonction du temps du mouvement cardiaque, et on les calcule en utilisant les ensembles de données d'image de navigation et les ensembles de données d'image.

8. Procédé suivant l'une des revendications précédentes, **caractérisé en ce que** l'on détermine et on corrige le mouvement respiratoire pour les deux directions de l'espace des ensembles de données d'image de navigation.

9. Installation (10) RM, qui est constituée pour la création d'images RM corrigées de respiration d'un volume à examiner d'une personne à examiner, lors d'un mouvement respiratoire de la personne à examiner, dans lequel le volume à examiner contient le cœur, dans laquelle l'installation RM a une unité (13) de commande et une unité (16) de mémoire, l'unité de mémoire mettant en mémoire des informations de commande réalisables par l'unité de commande, l'installation RM étant constituée pour effectuer, lors de la réalisation des informations de commande dans l'unité de commande, les stades suivants :
- enregistrement continu de signaux RM du volume à examiner pendant plusieurs cycles (23) cardiaques, dans lequel un cycle (23) cardiaque a plusieurs laps de temps (24, 25, 26) et est subdivisé en plusieurs phases cardiaques, dans lequel chaque phase cardiaque a plusieurs laps de temps,
dans lequel l'enregistrement continu comporte un enregistrement d'un ensemble de données d'image de navigation en 2D par cycle (23) cardiaque, pendant un laps de temps (24, 25, 26) du cycle (23) cardiaque, dans lequel on excite le volume à examiner et on remplit un espace de données brutes dans un modèle d'enregistrement cartésien de signaux RM, de manière à ce qu'une résolution spatiale dans deux de trois directions de l'espace du volume à examiner ait lieu, dans lequel, dans un premier cycle (23) cardiaque des plusieurs cycles (23) cardiaques, l'enregistrement de l'ensemble de données d'image de navigation en 2D a lieu dans un premier laps de temps (25) d'une phase cardiaque et, dans un deuxième cycle cardiaque des plusieurs cycles cardiaques, l'enregistrement de l'ensemble de données d'image de navigation en 2D a lieu dans une deuxième laps de temps (26) de la même phase cardiaque, dans lequel le premier laps de temps (25) est différent est différent du deuxième laps de temps (26), dans lequel l'enregistrement continu comporte un enregistrement de plusieurs ensembles de données d'image en 3D dans les autres laps de temps (24) du cycle (23) cardiaque, dans lequel, lors de l'enregistrement des ensembles de données d'image, on remplit un espace de données brutes dans un modèle d'enregistrement cartésien de signaux MR, de manière à ce qu'une résolution spatiale dans toutes les trois directions de l'espace du volume à examiner ait lieu,
- détermination du mouvement respiratoire à l'aide des ensembles de données d'image de navigation, qui ont été enregistrés dans les plusieurs cycles (23) cardiaques,
- correction du mouvement respiratoire déterminé dans les signaux RM enregistrés, pour la création des images RM corrigées de respiration.

10. Installation RM suivant la revendication 9, **caractérisée en ce que** l'installation RM est constituée pour effectuer, lors de l'exécution des informations de commande dans l'unité (13) de commande, un procédé suivant l'une des revendications 2 à 8.

11. Produit de programme d'ordinateur, qui comprend des moyens de programme et qui peut être chargé directement dans une unité (16) de mémoire d'une unité (13) de commande programmable d'une installation RM, pour effectuer tous les stades du procédé suivant l'une des revendications 1 à 8, lorsque les moyens de programme sont exécutés dans le dispositif de commande.

12. Support de données déchiffrables électroniquement, sur lequel sont mises en mémoire des informations de commande, qui peuvent être déchiffrées électroniquement et qui sont conformées de manière à ce qu'elles effectuent, lors de l'utilisation du support de données dans une unité (13) de commande d'une installation RM, le procédé suivant l'une des revendications 1 à 8, lorsque les informations de commande sont exécutées dans l'unité de commande.
